# EUROPEAN PATENT SPECIFICATION

(11) **EP 4 014 999 B1**
(45) Date of publication and mention of the grant of the patent: **29.11.2023**
(21) Application number: 20853232.5
(22) Date of filing: 30.07.2020
(51) Int. Cl.: A61K 31/366, A61K 31/7048, A61K 31/585, A61K 31/64, A61K 45/06, A61K 9/00, A61P 3/00, A61P 3/10, A61K 9/20

(54) **COMBINATION PRODUCT CONTAINING LIMONIN COMPOUND AND SULFONYLUREA DRUG**
KOMBINATIONSPRODUKT MIT LIMONINVERBINDUNG UND SULFONYLHARNSTOFFARZNEIMITTEL
PRODUIT DE COMBINAISON CONTENANT UN COMPOSÉ DE LIMONINE ET UN MÉDICAMENT À BASE DE SULFONYLURÉE

(30) Priority: 12.08.2019 CN 201910741989
(43) Date of publication of application: 22.06.2022
(73) Proprietor: ZHEJIANG YANGSHENGTANG INSTITUTE OF NATURAL MEDICATION CO., LTD., HANGZHOU, Zhejiang 310024 (CN)
(72) Inventor: LI, Dong, HANGZHOU, Zhejiang 310024 (CN); HAN, Quan, HANGZHOU, Zhejiang 310024 (CN); HU, Liu, HANGZHOU, Zhejiang 310024 (CN); XUE, Lian, HANGZHOU, Zhejiang 310024 (CN)
(74) Representative: Kolster Oy Ab
(86) International application number: PCT/CN2020/105728
(87) International publication number: WO 2021/027583

(56) References cited:
- EP-A1- 3 666 267
- CN-A- 102 481 276
- CN-A- 103 597 071
- CN-A- 108 210 566
- CN-A- 108 210 600
- CN-A- 109 381 471
- CN-A- 110 302 386
- JP-A- 2000 072 684
- US-A1- 2014 371 303
- US-B2- 9 610 276
- OVALLE-MAGALLANES BERENICE ET AL: "Multi-target antidiabetic mechanisms of mexicanolides fromSwietenia humilis", PHYTOMEDICINE, vol. 58, 13 March 2019 (2019-03-13), XP085683898, ISSN: 0944-7113, DOI: 10.1016/J.PHYMED.2019.152891
- HORIBA TARO ET AL: "Dietary Obacunone Supplementation Stimulates Muscle Hypertrophy，and Suppresses Hyperglycemia and Obesity through the TGR5 and PPARγ Pathway", BIOCHEMICAL AND BIOPHYSICAL RESEARCH COMMUNICATIONS, 5 June 2015 (2015-06-05), pages 846-852, XP055780524,
- HANSEN R A ET AL: "A retrospective cohort study of economic outcomes and adherence to monotherapy with metformin, pioglitazone, or a sulfonylurea among patients with type 2 diabetes mellitus in the united states from 2003 to 2005", CLINICAL THERAPEUTICS, ELSEVIER, AMSTERDAM, NL, vol. 32, no. 7, 1 July 2010 (2010-07-01), pages 1308-1319, XP027185915, ISSN: 0149-2918 [retrieved on 2010-07-30]
- Gualdani Roberta, Cavalluzzi Maria, Lentini Giovanni, Habtemariam Solomon: "The Chemistry and Pharmacology of Citrus Limonoids", Molecules, vol. 21, no. 11 , page 1530, XP055780471, DOI: 10.3390/molecules21111530
- S. A. Mohammed, A. G. Yaqub, K. A. Sanda, A. O. Nicholas, W. Arastus, M. Muhammad and S. Abdullahi: "Review on diabetes, synthetic drugs and glycemic effects of medicinal plants", Journal of Medicinal Plants Research, vol. 7, no. 36, 25 September 2013 (2013-09-25), pages 2628-2637, XP009526005, DOI: 10.5897/JMPR2013.5169
- Yan Min, Zhou Yu; He Xiao-Han; Mei Ming-Xin; Dong Quan: "FOOD AND FEï¼²MENTATION INDUSTï¼²IES", Food and Fermentation Industries, vol. 44, no. 2, 1 January 2018 (2018-01-01), pages 290-296, XP055780486, DOI: 10.13995/j.cnki.11-1802/ts.014827

## Description

### Technical Field

The present invention belongs to the technical field of medicine, and specifically relates to a combination product comprising a limonoid compound (and a pharmaceutically acceptable salt thereof) and a sulfonylurea compound The present invention also relates to the combination product for use in the treatment and/or prevention of a disease associated with diabetes and metabolic syndrome.

### Background Art

According to IDF statistics, there were about 425 million people with diabetes worldwide in 2017, i.e., 1 out of every 11 people has diabetes. The number of diabetic patients in China is about 110 million, ranking first in the world. It is predicted that by 2040, 642 million people worldwide will have diabetes, and the diabetic patients in China will reach 151 million. Diabetes requires life-long monitoring and treatment, and if not being well controlled, it will lead to secondary cardiovascular diseases, blindness, stroke, diabetic nephropathy, diabetic gangrene and other complications in patients, which will seriously endanger human health and life.

More than 90% of diabetes is type II diabetes, and oral hypoglycemic agents are the main treatment method. At present, the main oral hypoglycemic drugs include: sulfonylureas, biguanides, α-glucosidase inhibitors, thiazolidinediones, DPP-4 inhibitors, etc., but the oral hypoglycemic drugs are prone to severe side effects such as drug resistance, low blood glucose, and toxicity to liver and kidney.

Sulfonylurea compounds (SU) are the earliest, most diverse, and most widely used oral hypoglycemic drugs. SU drugs are divided into the first and second generations. The glimepiride developed in recent years is called the third-generation SU drug due to small dosage, certain effect of improving insulin resistance, and reducing insulin dosage; examples thereof include: glibenclamide, gliclazide, glipizide, gliquidone and glimepiride and so on. The mechanism action thereof is: (1) effect on pancreatic islet β cells: it is known that when SU exerts its effect on pancreatic β cells, it must first bind to a SU receptor on the surface of the β cell, and then couples to the ATP-sensitive potassium channel on the surface of the β cell to close the channel, then depolarize the cell membrane, thereby releasing insulin; therefore, SU can stimulate β cells to release insulin, thereby reducing blood glucose; (2) effect on pancreatic outside: SU can promote the synthesis of liver glycogen, reduce the production of glycogen, and slow the release rate of liver glucose into blood; at the same time, SU can increase the uptake and utilization of glucose by surrounding tissues, and increase the number of insulin receptors on the cell membrane, thereby increasing the body's insulin sensitivity. The main side effects of sulfonylurea compounds are: risk of hypoglycemia when using at high-dose (especially in elderly patients and patients with liver and kidney insufficiency), blood system reactions (reduction of leukocytes, neutrophils, platelets or whole blood cells, hemolytic anemia, etc.), allergic reactions (skin pruritus, urticaria, erythema, dermatitis, etc.), nervous system reactions (headache, dizziness, paresthesia, drowsiness, tinnitus, vision loss, tremor, ataxia, etc.).

The limonoid compounds are mainly present in fruits of rutaceous plants, such as immature bitter orange, navel orange, citrus reticulate, fragrant citrus, pomelo and the like. Their contents are higher in the cores (seeds), and lower in the peel (about 1/10,000 to 5/100,000). About 50 kinds of limonoid compounds have been isolated and identified from citrus plants. The limonoid compounds have various biological activities such as antitumor, insect antifeedant, antiviral, analgesic, anti-inflammatory and hypnotic, and can be used in functional food additives, anti-cancer foods, pesticides, feed additives, etc.

Considering the hypoglycemic effect and side effects of sulfonylurea compounds, it is urgent to find a pharmaceutical composition product that is simple to take, good in effect, and low in side effects.

EP 3 666 267 discloses a combination product comprising a limonoid compound and a biguanide compound for use in the prevention and/or treatment of diseases associated with diabetes and metabolic syndrome.

### Contents of the Invention

Note that the references to methods of treatment in this description are to be interpreted as references to the compounds, pharmaceutical compositions and medicaments of the present invention for use in a method for treatment of the human (or animal) body by therapy.

The present invention provides a combination product comprising a limonoid compound (or a pharmaceutically acceptable salt thereof) and a sulfonylurea compound and a use of this composition for preventing or/and treating a disease associated with diabetes and metabolic syndrome. Compared with sulfonylurea compound or limonoid compound (or a pharmaceutically acceptable salt thereof) as monotherapy at the same dose, the combination product containing a limonoid compound (or a pharmaceutically acceptable salt thereof) and a sulfonylurea compound as mentioned in the present invention can significantly enhance therapeutic effects such as hypoglycemic effect, and show synergistic effect. At the same time, the amount of sulfonylurea compound is reduced, thereby reducing its side effects.

In a first aspect of the present invention, there is provided a combination product comprising a limonoid compound (or a pharmaceutically acceptable thereof), and a sulfonylurea compound, or a combination product comprising only a limonoid compound (or a pharmaceutically acceptable salt thereof), and a sulfonylurea compound as active ingredients

The limonoid compound as mentioned in the present invention is a general term for a class of highly oxidized compounds with a 4,4,8-trimethyl-17-furanosteroid skeleton or derivatives thereof (or can be expressed as compounds consisting of variants of furanolactone polycyclic core structure, and having four fused 6-membered rings and one furan ring). Specifically, the limonoid compound is one or more selected from the group consisting of limonin, isolimonic acid, limonin glycoside, isolimonic acid glycoside, obacunone, isoobacunoic acid, obacunone glycoside, ichangin, ichangensin, ichangin glycoside, nomilin, nomilinic acid, deacetylnomilin, nomilin glycoside, deacetylnomilin glycoside and nomilinic acid glycoside. The structural formula of an exemplary limonoid compound, i.e., limonin, is shown below.

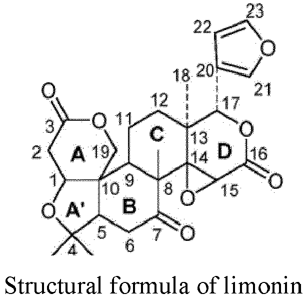

Further, the glucoside derivatives of the limonoid compound as mentioned in the present invention include, but are not limited to: limonin 17-β-glucopyranoiside, ichangin 17-β-D-glucopyranoiside, isolimonic acid 17-β-D-glucopyranoside, deacetylnomilin 17-β-D-glucopyranoside, nomilin 17-β-D-glucopyranoside, obacunone 17-β-D-glucopynoside, nomilinic acid 17-β-D-glucopyranosid,

In some embodiments, the limonoid compound as mentioned in the present invention is in the form of a monomer or an extract. The monomer is extracted or artificially synthesized, and its sources may be commercially available, or they can be easily prepared and obtained by the prior art in the art.

The sulfonylurea compounds mentioned in the present invention are selected from the group consisting of glibenclamide, gliclazide, glipizide, gliquidone, glimepiride.

In some embodiments, the combination product is in the form of a pharmaceutical composition, and the pharmaceutical composition is in a unit dosage form.

In some embodiments, the limonoid compound (or a pharmaceutically acceptable salt thereof) and the sulfonylurea compound are each in the form of a separate preparation. Further, the limonoid compound (or a pharmaceutically acceptable salt thereof) and the sulfonylurea compound are each in the form of a separate unit dose. Further, the limonoid compound (or a pharmaceutically acceptable salt thereof) and the sulfonylurea compound can be administered simultaneously or sequentially.

In some embodiments, the sulfonylurea compound has an amount of 10 mg, 20 mg, 40 mg, 50 mg, 75 mg, 100 mg, 150 mg, 200 mg, 250 mg, 300 mg, 375 mg, 500 mg, 750 mg, 1500 mg, 1875 mg, or 2000 mg, and the ranges between these amounts, wherein the ranges include but are not limited to: 10 mg to 20 mg, 10 mg to 40 mg, 10 mg to 50 mg, 10 mg to 75 mg, 10 mg to 100 mg, 10 mg to 150 mg, 10 mg to 200 mg, 10 mg to 250 mg, 10 mg to 300 mg, 10 mg to 375 mg, 10 mg to 500 mg, 10 mg to 750 mg, 10 mg to 1500 mg, 10 mg to 1875 mg, 10 mg to 2000 mg, 20 mg to 40 mg, 20 mg to 50 mg, 20 mg to 75 mg, 20 mg to 100 mg, 20 mg to 150 mg, 20 mg to 200 mg, 20 mg to 250 mg, 20 mg to 300 mg, 20 mg to 375 mg, 20 mg to 500 mg, 20 mg to 750 mg, 20 mg to 1500 mg, 20 mg to 1875 mg, 20 mg to 2000 mg, 40 mg to 50 mg, 40 mg to 75 mg, 40 mg to 100 mg, 40 mg to 150 mg, 40 mg to 200 mg, 40 mg to 250 mg, 40 mg to 300 mg, 40 mg to 375 mg, 40 mg to 500 mg, 40 mg to 750 mg, 40 mg to 1500 mg, 40 mg to 1875 mg, 40 mg to 2000 mg, 50 mg to 75 mg, 50 mg to 100 mg, 50 mg to 150 mg, 50 mg to 200 mg, 50 mg to 250 mg, 50 mg to 300 mg, 50 mg to 375 mg, 50 mg to 500 mg, 50 mg to 750 mg, 50 mg to 1500 mg, 50 mg to 1875 mg, 50 mg to 2000 mg, 75 mg to 100 mg, 75 mg to 150 mg, 75 mg to 200 mg, 75 mg to 250 mg, 75 mg to 300 mg, 75 mg to 375 mg, 75 mg to 500 mg, 75 mg to 750 mg, 75 mg to 1500 mg, 75 mg to 1875 mg, 75 mg to 2000 mg, 100 mg to 150 mg, 100 mg to 200 mg, 100 mg to 250 mg, 100 mg to 300 mg, 100 mg to 375 mg, 100 mg to 500 mg, 100 mg to 750 mg, 100 mg to 1500 mg, 100 mg to 1875 mg, 100 mg to 2000 mg, 150 mg to 200 mg, 150 mg to 250 mg, 150 mg to 300 mg, 150 mg to 375 mg, 150 mg to 500 mg, 150 mg to 750 mg, 150 mg to 1500 mg, 150 mg to 1875 mg, 150 mg to 2000 mg, 200 mg to 250 mg, 200 mg to 300 mg, 200 mg to 375 mg, 200 mg to 500 mg, 200 mg to 750 mg, 200 mg to 1500 mg, 200 mg to 1875 mg, 200 mg to 2000 mg, 250 mg to 300 mg, 250 mg to 375 mg, 250 mg to 500 mg, 250 mg to 750 mg, 250 mg to 1500 mg, 250 mg to 1875 mg, 250 mg to 2000 mg, 300 mg to 375 mg, 300 mg to 500 mg, 300 mg to 750 mg, 300 mg to 1500 mg, 300 mg to 1875 mg, 300 mg to 2000 mg, 375 mg to 500 mg, 375 mg to 750 mg, 375 mg to 1500 mg, 375 mg to 1875 mg, 375 mg to 2000 mg, 500 mg to 750 mg, 500 mg to 1500 mg, 500 mg to 1875 mg, 500 mg to 2000 mg, 750 mg to 1500 mg, 750 mg to 1875 mg, 750 mg to 2000 mg, and 1875 mg to 2000 mg.

In some embodiments, the limonoid compound (or a pharmaceutically acceptable salt thereof) has an amount of 10 mg, 20 mg, 40 mg, 50 mg, 75 mg, 100 mg, 150 mg, 200 mg, 250 mg, 300 mg, 375 mg, 500 mg, 750 mg, 1500 mg, 1875 mg, or 2000 mg, and the ranges between these amounts, wherein the ranges include but are not limited to: 10 mg to 20 mg, 10 mg to 40 mg, 10 mg to 50 mg, 10 mg to 75 mg, 10 mg to 100 mg, 10 mg to 150 mg, 10 mg to 200 mg, 10 mg to 250 mg, 10 mg to 300 mg, 10 mg to 375 mg, 10 mg to 500 mg, 10 mg to 750 mg, 10 mg to 1500 mg, 10 mg to 1875 mg, 10 mg to 2000 mg, 20 mg to 40 mg, 20 mg to 50 mg, 20 mg to 75 mg, 20 mg to 100 mg, 20 mg to 150 mg, 20 mg to 200 mg, 20 mg to 250 mg, 20 mg to 300 mg, 20 mg to 375 mg, 20 mg to 500 mg, 20 mg to 750 mg, 20 mg to 1500 mg, 20 mg to 1875 mg, 20 mg to 2000 mg, 40 mg to 50 mg, 40 mg to 75 mg, 40 mg to 100 mg, 40 mg to 150 mg, 40 mg to 200 mg, 40 mg to 250 mg, 40 mg to 300 mg, 40 mg to 375 mg, 40 mg to 500 mg, 40 mg to 750 mg, 40 mg to 1500 mg, 40 mg to 1875 mg, 40 mg to 2000 mg, 50 mg to 75 mg, 50 mg to 100 mg, 50 mg to 150 mg, 50 mg to 200 mg, 50 mg to 250 mg, 50 mg to 300 mg, 50 mg to 375 mg, 50 mg to 500 mg, 50 mg to 750 mg, 50 mg to 1500 mg, 50 mg to 1875 mg, 50 mg to 2000 mg, 75 mg to 100 mg, 75 mg to 150 mg, 75 mg to 200 mg, 75 mg to 250 mg, 75 mg to 300 mg, 75 mg to 375 mg, 75 mg to 500 mg, 75 mg to 750 mg, 75 mg to 1500 mg, 75 mg to 1875 mg, 75 mg to 2000 mg, 100 mg to 150 mg, 100 mg to 200 mg, 100 mg to 250 mg, 100 mg to 300 mg, 100 mg to 375 mg, 100 mg to 500 mg, 100 mg to 750 mg, 100 mg to 1500 mg, 100 mg to 1875 mg, 100 mg to 2000 mg, 150 mg to 200 mg, 150 mg to 250 mg, 150 mg to 300 mg, 150 mg to 375 mg, 150 mg to 500 mg, 150 mg to 750 mg, 150 mg to 1500 mg, 150 mg to 1875 mg, 150 mg to 2000 mg, 200 mg to 250 mg, 200 mg to 300 mg, 200 mg to 375 mg, 200 mg to 500 mg, 200 mg to 750 mg, 200 mg to 1500 mg, 200 mg to 1875 mg, 200 mg to 2000 mg, 250 mg to 300 mg, 250 mg to 375 mg, 250 mg to 500 mg, 250 mg to 750 mg, 250 mg to 1500 mg, 250 mg to 1875 mg, 250 mg to 2000 mg, 300 mg to 375 mg, 300 mg to 500 mg, 300 mg to 750 mg, 300 mg to 1500 mg, 300 mg to 1875 mg, 300 mg to 2000 mg, 375 mg to 500 mg, 375 mg to 750 mg, 375 mg to 1500 mg, 375 mg to 1875 mg, 375 mg to 2000 mg, 500 mg to 750 mg, 500 mg to 1500 mg, 500 mg to 1875 mg, 500 mg to 2000 mg, 750 mg to 1500 mg, 750 mg to 1875 mg, 750 mg to 2000 mg, and 1875 mg to 2000 mg.

The sulfonylurea compound is selected from glibenclamide, gliclazide, glipizide, gliquidone, glimepiride and the limonoid compound is one or more selected from: limonin, isolimonic acid, limonin glycoside, isolimonic acid glycoside, obacunone, isoobacunoic acid, obacunone glycoside, ichangin, ichangensin, ichangin glycoside, nomilin, nomilinic acid, deacetylnomilin, nomilin glycoside, deacetylnomilin glycoside and nomilinic acid glycoside.

In some embodiments, the combination product further comprises a pharmaceutically acceptable carrier, diluent, or excipient.

In some embodiments, the combination product is in the form of tablet, capsule, granule, syrup, powder, lozenge, sachet, cachet, elixir, suspension, emulsion, solution, syrup, aerosol, ointment, cream and injection.

In a second aspect of the present invention, there is provided a use of the combination product in manufacture of a medicament for the prevention and/or treatment of a disease associated with diabetes and metabolic syndrome. In some embodiments, the diabetes is type I diabetes. In some embodiments, the diabetes is type II diabetes.

In a third aspect of the present invention, there is provided a method of administering the limonoid compound (or a pharmaceutically acceptable salt thereof) and the sulfonylurea compound in combination to prevent and/or treat a disease. In some embodiments, there is provided a method of administering the limonoid compound (or a pharmaceutically acceptable salt thereof) and the sulfonylurea compound in combination to prevent and/or treat a disease associated with diabetes and metabolic syndrome. In some embodiments, there is provided a method of administering the limonoid compound (or a pharmaceutically acceptable salt thereof) and the sulfonylurea compound in combination to lower a blood glucose. In some embodiments, there is provided a method of administering the limonoid compound (or a pharmaceutically acceptable salt thereof) and the sulfonylurea compound in combination to improve an insulin sensitivity. In some embodiments, there is provided a method of administering the limonoid compound (or a pharmaceutically acceptable salt thereof) and the sulfonylurea compound in combination to improve a leptin sensitivity.

In some embodiments, the limonoid compound (or a pharmaceutically acceptable salt thereof) and the sulfonylurea compound can be mixed into a preparation and administered in the form of a pharmaceutical composition (preferably, a dosage unit form); in some embodiments, the limonoid compound (or a pharmaceutically acceptable salt thereof) and the sulfonylurea compound are each in separate preparation form (preferably, each in separate dosage unit form) and separately administered; in some embodiments, the limonoid compound (or a pharmaceutically acceptable salt thereof) and the sulfonylurea compound are administered simultaneously; in some embodiments, the limonoid compound (or a pharmaceutically acceptable salt thereof) and the sulfonylurea compound are administered one after another; in some embodiments, the limonoid compound (or a pharmaceutically acceptable salt thereof) and the sulfonylurea compound are administered one after another at a time interval of about 30 minutes, or about 1 hour, or about 2 hours, or about 4 hours, or about 8 hours, or about 12 hours. In some embodiments, as required, the combination product comprising the limonoid compound (or a pharmaceutically acceptable salt thereof) and the sulfonylurea compound according to the present invention that is in the form of pharmaceutical composition is administered for (preferably, a dosage unit form), including, but are not limited to: 1, 2, 3, 4, 5 or 6 times per day. In some embodiments, as required, the combination product comprising the limonoid compound (or a pharmaceutically acceptable salt thereof) and the sulfonylurea compound according to the present invention that are each in separate preparation form (preferably, each in separate dosage unit form) is administered for, including, but are not limited to: 1, 2, 3, 4, 5 or 6 times per day.

In some embodiments, the limonin compound (or a pharmaceutically acceptable salt thereof), and the sulfonylurea compound have a daily dosage as follows: as calculated according to adult body weight of 60kg, the daily dosage of the sulfonylurea compound is 10 mg, 20 mg, 40 mg, 50 mg, 75 mg, 100 mg, 150 mg, 200 mg, 250 mg, 300 mg, 375 mg, 500 mg, 750 mg, 1500 mg, 1875 mg or 2000 mg, and the ranges between these dosages, wherein the ranges include but are not limited to: 10 mg to 20 mg, 10 mg to 40 mg, 10 mg to 50 mg, 10 mg to 75 mg, 10 mg to 100 mg, 10 mg to 150 mg, 10 mg to 200 mg, 10 mg to 250 mg, 10 mg to 300 mg, 10 mg to 375 mg, 10 mg to 500 mg, 10 mg to 750 mg, 10 mg to 1500 mg, 10 mg to 1875 mg, 10 mg to 2000 mg, 20 mg to 40 mg, 20 mg to 50 mg, 20 mg to 75 mg, 20 mg to 100 mg, 20 mg to 150 mg, 20 mg to 200 mg, 20 mg to 250 mg, 20 mg to 300 mg, 20 mg to 375 mg, 20 mg to 500 mg, 20 mg to 750 mg, 20 mg to 1500 mg, 20 mg to 1875 mg, 20 mg to 2000 mg, 40 mg to 50 mg, 40 mg to 75 mg, 40 mg to 100 mg, 40 mg to 150 mg, 40 mg to 200 mg, 40 mg to 250 mg, 40 mg to 300 mg, 40 mg to 375 mg, 40 mg to 500 mg, 40 mg to 750 mg, 40 mg to 1500 mg, 40 mg to 1875 mg, 40 mg to 2000 mg, 50 mg to 75 mg, 50 mg to 100 mg, 50 mg to 150 mg, 50 mg to 200 mg, 50 mg to 250 mg, 50 mg to 300 mg, 50 mg to 375 mg, 50 mg to 500 mg, 50 mg to 750 mg, 50 mg to 1500 mg, 50 mg to 1875 mg, 50 mg to 2000 mg, 75 mg to 100 mg, 75 mg to 150 mg, 75 mg to 200 mg, 75 mg to 250 mg, 75 mg to 300 mg, 75 mg to 375 mg, 75 mg to 500 mg, 75 mg to 750 mg, 75 mg to 1500 mg, 75 mg to 1875 mg, 75 mg to 2000 mg, 100 mg to 150 mg, 100 mg to 200 mg, 100 mg to 250 mg, 100 mg to 300 mg, 100 mg to 375 mg, 100 mg to 500 mg, 100 mg to 750 mg, 100 mg to 1500 mg, 100 mg to 1875 mg, 100 mg to 2000 mg, 150 mg to 200 mg, 150 mg to 250 mg, 150 mg to 300 mg, 150 mg to 375 mg, 150 mg to 500 mg, 150 mg to 750 mg, 150 mg to 1500 mg, 150 mg to 1875 mg, 150 mg to 2000 mg, 200 mg to 250 mg, 200 mg to 300 mg, 200 mg to 375 mg, 200 mg to 500 mg, 200 mg to 750 mg, 200 mg to 1500 mg, 200 mg to 1875 mg, 200 mg to 2000 mg, 250 mg to 300 mg, 250 mg to 375 mg, 250 mg to 500 mg, 250 mg to 750 mg, 250 mg to 1500 mg, 250 mg to 1875 mg, 250 mg to 2000 mg, 300 mg to 375 mg, 300 mg to 500 mg, 300 mg to 750 mg, 300 mg to 1500 mg, 300 mg to 1875 mg, 300 mg to 2000 mg, 375 mg to 500 mg, 375 mg to 750 mg, 375 mg to 1500 mg, 375 mg to 1875 mg, 375 mg to 2000 mg, 500 mg to 750 mg, 500 mg to 1500 mg, 500 mg to 1875 mg, 500 mg to 2000 mg, 750 mg to 1500 mg, 750 mg to 1875 mg, 750 mg to 2000 mg and 1875 mg to 2000 mg. As calculated according to adult body weight of 60kg, the daily dosage of the limonoid compound (or a pharmaceutically acceptable salt thereof) is 10 mg, 20 mg, 40 mg, 50 mg, 75 mg, 100 mg, 150 mg, 200 mg, 250 mg, 300 mg, 375 mg, 500 mg, 750 mg, 1500 mg, 1875 mg or 2000 mg, and the ranges of between these dosages, wherein the ranges includes but are not limited to: 10 mg to 20 mg, 10 mg to 40 mg, 10 mg to 50 mg, 10 mg to 75 mg, 10 mg to 100 mg, 10 mg to 150 mg, 10 mg to 200 mg, 10 mg to 250 mg, 10 mg to 300 mg, 10 mg to 375 mg, 10 mg to 500 mg, 10 mg to 750 mg, 10 mg to 1500 mg, 10 mg to 1875 mg, 10 mg to 2000 mg, 20 mg to 40 mg, 20 mg to 50 mg, 20 mg to 75 mg, 20 mg to 100 mg, 20 mg to 150 mg, 20 mg to 200 mg, 20 mg to 250 mg, 20 mg to 300 mg, 20 mg to 375 mg, 20 mg to 500 mg, 20 mg to 750 mg, 20 mg to 1500 mg, 20 mg to 1875 mg, 20 mg to 2000 mg, 40 mg to 50 mg, 40 mg to 75 mg, 40 mg to 100 mg, 40 mg to 150 mg, 40 mg to 200 mg, 40 mg to 250 mg, 40 mg to 300 mg, 40 mg to 375 mg, 40 mg to 500 mg, 40 mg to 750 mg, 40 mg to 1500 mg, 40 mg to 1875 mg, 40 mg to 2000 mg, 50 mg to 75 mg, 50 mg to 100 mg, 50 mg to 150 mg, 50 mg to 200 mg, 50 mg to 250 mg, 50 mg to 300 mg, 50 mg to 375 mg, 50 mg to 500 mg, 50 mg to 750 mg, 50 mg to 1500 mg, 50 mg to 1875 mg, 50 mg to 2000 mg, 75 mg to 100 mg, 75 mg to 150 mg, 75 mg to 200 mg, 75 mg to 250 mg, 75 mg to 300 mg, 75 mg to 375 mg, 75 mg to 500 mg, 75 mg to 750 mg, 75 mg to 1500 mg, 75 mg to 1875 mg, 75 mg to 2000 mg, 100 mg to 150 mg, 100 mg to 200 mg, 100 mg to 250 mg, 100 mg to 300 mg, 100 mg to 375 mg, 100 mg to 500 mg, 100 mg to 750 mg, 100 mg to 1500 mg, 100 mg to 1875 mg, 100 mg to 2000 mg, 150 mg to 200 mg, 150 mg to 250 mg, 150 mg to 300 mg, 150 mg to 375 mg, 150 mg to 500 mg, 150 mg to 750 mg, 150 mg to 1500 mg, 150 mg to 1875 mg, 150 mg to 2000 mg, 200 mg to 250 mg, 200 mg to 300 mg, 200 mg to 375 mg, 200 mg to 500 mg, 200 mg to 750 mg, 200 mg to 1500 mg, 200 mg to 1875 mg, 200 mg to 2000 mg, 250 mg to 300 mg, 250 mg to 375 mg, 250 mg to 500 mg, 250 mg to 750 mg, 250 mg to 1500 mg, 250 mg to 1875 mg, 250 mg to 2000 mg, 300 mg to 375 mg, 300 mg to 500 mg, 300 mg to 750 mg, 300 mg to 1500 mg, 300 mg to 1875 mg, 300 mg to 2000 mg, 375 mg to 500 mg, 375 mg to 750 mg, 375 mg to 1500 mg, 375 mg to 1875 mg, 375 mg to 2000 mg, 500 mg to 750 mg, 500 mg to 1500 mg, 500 mg to 1875 mg, 500 mg to 2000 mg, 750 mg to 1500 mg, 750 mg to 1875 mg, 750 mg to 2000 mg and 1875 mg to 2000 mg.

In a fourth aspect of the present invention, there is provided a method for preparing a combination product in the form of a pharmaceutical composition. In order to improve its operability as a drug or its absorbability when used in a living body, the limonoid compound or a pharmaceutically acceptable salt thereof and the sulfonylurea compound are preferably combined with a pharmaceutical adjuvant such as a pharmaceutically acceptable carrier, excipient, diluent, etc., so as to form a preparation, thereby obtaining the form.

In a fifth aspect of the invention, there is provided a kit, the kit comprising the combination product described herein.

The term "pharmaceutically acceptable salt" as used throughout this description refers to a salt of a free acid or a free base, that is typically prepared by reacting the free base with a suitable organic or inorganic acid or by reacting the acid with a suitable organic or inorganic base. The term can be used for any compound, including limonoid compounds (having the function of free acid or free base) and the like. Representative salts include: acetate, benzenesulfonate, benzoate, bicarbonate, bisulfate, hydrogen tartrate, borate, bromide, calcium edetate, camphorsulfonate, carbonate, chloride, clavulanate, citrate, dihydrochloride, edetate, ethanedisulfonate, estolate, esylate, fumarate, glucoheptonate, gluconate, glutamate, glycol lylarsanilate, hexylresorcinate, hydrabamine, hydrobromide, hydrochloride, hydroxynaphthoate, iodide, isethionate, lactate, lactobionate, laurate, malate, maleate, mandelate, methanesulfonate, methobromate), methonitrate, methosulfate, monopotassium maleate, mucate, naphthalenesulfonate, nitrate, N-methylglucosamine salt, oxalate, pamoate, palmitate, pantothenate, phosphate/bisphosphate, polygalacturonate, potassium salt, salicylate, sodium salt, stearate, subacetate, succinate, tannate, tartrate, teoclate, p-toluenesulfonate, triethiodide, trimethylamine salt, and valerate. When an acidic substituent is present, for example, -COOH, an ammonium salt, morpholine salt, sodium salt, potassium salt, barium salt, calcium salt, and the like can be formed for use in a dosage form. When a basic group, for example an amino group or a basic heteroaryl group such as pyridyl, is present, an acidic salt such as a hydrochloride, hydrobromide, phosphate, sulfate, trifluoroacetate, trichloroacetate, acetate, oxalate, maleate, pyruvate, malonate, succinate, citrate, tartrate, fumarate, mandelate, benzoate, cinnamate, mesylate, ethanesulfonate, picrate, etc.

The sources of sulfonylurea compounds referred to in the present invention may include, : glibenclamide single tablets/capsules, gliclazide single tablets/capsules, glipizide single tablets/capsules, gliquidone single tablets/capsules, glimepiride single tablets/capsules, glibenclamide/metformin tablets/capsules, gliclazide/metformin tablets/capsules, glipizide/metformin tablets/capsules, gliquidone/metformin tablets/capsules, glimepiride/metformin tablets/capsules, pioglitazone hydrochloride/glimepiride tablets, glimepiride/rosiglitazone maleate tablets.

### Specific Models for Carrying Out the Invention

The present invention is further described below through specific examples and comparative examples. However, it should be understood that these examples and comparative examples are only used for more detailed and specific explanation, and should not be understood as limiting the present invention in any form.

In the examples of the present invention, the following diabetic mouse models (the models were well known to those skilled in the art or were easily available according to conventional textbooks, technical manuals, and scientific literature in the art) were used to simulate the pathological conditions of different stages of diabetes in humans. The limonoid compound mentioned in the examples was present in the form of a monomer or an extract. The monomer was extracted or artificially synthesized, and its sources were commercially available, or it could be easily prepared and obtained by the prior art in the art.

### Example 1

### Effects of limonoid compound, glibenclamide or combination thereof on blood glucose in mouse pancreatic islet β-cell injury model

In this example, a mouse pancreatic islet β-cell injury model was established by modeling ICR mice with streptozotocin (STZ) (Li Nan et al., Protective effect of pine pollen on kidney damage in diabetic nephropathy mice, Science and Technology Review, 2014, 32 (4/5): 95-99), and used to complete the evaluation of hypoglycemic effect in animals (this model could simulate pancreatic islet β-cell damage state of type I and type II diabetics). The limonoid compound was selected from the group consisting of limonin, isolimonic acid, limonin glycoside, and isolimonic acid glycoside, and glibenclamide single administration group, limonin single administration group, isolimonic acid single administration group, limonin glycoside singe administration group, isolimonic acid glycoside singe administration group, and respective combination thereof with glibenclamide administration groups were set.

Conditions of experimental feeding: ICR mice (20 ± 2g), aged 6 weeks, purchased from Zhejiang Academy of Medical Sciences, and subjected to experimental feeding after 7 days of preliminary feeding. It should be noted that the conditions for raising the mice were as follows: the temperature was 23 ± 1 °C, the humidity was 55 ± 10%, the lights were turned on between 7 am and 7 pm (the lights were turned off at other time), and the mice were allowed to freely take in water and feed. The experimental feed was mouse growth-stable feed (GB M2118), and the daily feeding and management of the animals were under the responsibility of the animal security department, which provided the animals with sufficient diet and fresh drinking water daily.

Experimental grouping: 15 male mice were randomly selected as the normal control group. After fasting for 12 hours, the remaining mice were intraperitoneally injected once with STZ at a dose of 150 mg/kg, and 72 hours later, the mice with blood glucose value of 15 to 25 mmol/L were undifferentiatedly grouped and used in the experiment, 15 animals in each group, and subjected to blood sampling and detection of indicators after two weeks of administration.

Gavage doses: the gavage dose was 0.02 g/kg per day for the limonin group, the gavage dose was 0.02 g/kg per day for the isolimonic acid group, the gavage dose was 0.02 g/kg per day for the limonin glycoside group, the gavage dose was 0.02 g/kg per day for the isolimonic acid glycoside group, the gavage dose of glibenclamide was 0.02 g/kg per day for the glibenclamide group, limonin at a dose of 0.01 g/kg and glibenclamide at a dose of 0.01 g/kg were simultaneously gavaged per day for the limonin/glibenclamide combination group, isolimonic acid at a dose of 0.01 g/kg and glibenclamide at a dose of 0.01 g/kg were simultaneously gavaged per day for the isolimonic acid/glibenclamide combination group, limonin glycoside at a dose of 0.01 g/kg and glibenclamide at a dose of 0.01 g/kg were simultaneously gavaged per day for the limonin glycoside/glibenclamide combination group, isolimonic acid glycoside at a dose of 0.01 g/kg and glibenclamide at a dose of 0.01 g/kg were simultaneously gavaged per day for the isolimonic acid glycoside/glibenclamide combination group, the gavage volume was 10 mL/kg, and the normal group and the model group were administrated with 10 mL/kg of distilled water. Two weeks later, the blood glucose values were measured by tail trimming method (Johnson's blood glucose meter) 1 h after the last administration, and the average of each group was obtained. SPSS 16.0 software was used for statistical analysis. The data were expressed as mean and standard deviation. The data before and after were analyzed by t-test, and P <0.05 was considered statistically significant. The test results were shown in Table 1 below.

**Table 1: Blood glucose values of STZ mice after two weeks of daily intragastric gavage.**

| Group | Formulation and dosage of administration | Blood glucose value (mmol/L) |
|---|---|---|
| Normal control group | None | 7.5±0.6 |
| Model group | None | 29.5±4.6 |
| Glibenclamide group | Glibenclamide 0.02g/kg | 18.3±3.2** |
| Limonin group | Limonin 0.02g/kg | 19.5±1.8** |
| Isolimonic acid group | Isolimonic acid 0.02g/kg | 18.2±3.1** |
| Limonin glycoside group | Limonin glycoside 0.02g/kg | 18.9±2.2** |
| Isolimonic acid glycoside group | Isolimonic acid glycoside 0.02g/kg | 17.9±2.8** |
| Limonin combination group | Limonin 0.01 g/kg + Glibenclamide 0.01 g/kg | 7.8±1.0** |
| Isolimonic acid combination group | Isolimonic acid 0.01 g/kg + Glibenclamide 0.01 g/kg | 8.0±1.7** |
| Limonin glycoside combination | Limonin glycoside 0.01 g/kg + Glibenclamide 0.01 g/kg | 8.2±1.3** |
| Isolimonic acid glycoside combination group | Isolimonic acid glycoside 0.01 g/kg + Glibenclamide 0.01 g/kg | 8.4±2.0** |

| | | |
|---|---|---|
| Note *: After independent t-test, compared with the model group, the difference was extremely significant (P <0.05) **: After independent t-test, compared with the model group, the difference was extremely significant (P <0.01) | | |

### Discussion of experimental results

From the above results, it could be seen that, compared with the model group, either in single administration or in combination administration with glibenclamide, limonin and its derivatives could significantly reduce the blood glucose values of the mice with STZ islet cell injury. The administration of limonin and its derivatives in combination with glibenclamide had significantly improved the effect as compared with their single administration, showing a synergistic effect. In addition, when limonin and its derivatives were administrated in combination with glibenclamide, as compared with their single administration, the doses of both could be effectively reduced while comparable glucose-lowering effects could still be achieved, which improved the safety of therapeutic regimen and reduced side effects.

### Example 2

### Effects of limonoid compound, gliclazide or combination thereof on blood glucose and leptin in mouse type II diabetes model

In the present example, db/db mice (line name BKS.Cg-Dock7^{m+/+}Lepr^{db}/Nju) were used to perform hypoglycemic efficacy evaluation test of animals (blood glucose level and leptin). The limonoid compound was selected from obacunone, isoobacunoic acid and obacunone glycoside, and gliclazide single administration group, obacunone single administration group, isoobacunoic acid single administration group, obacunone glycoside single administered group, and respective combination thereof with gliclazide administration groups were set.

Conditions for experimental feeding: as type II diabetes model mice, 6-week-old SPF-grade db/db mice were purchased from the Nanjing Model Biology Institute, and subjected to experimental feeding after 7 days of preliminary feeding. It should be noted that the conditions for raising the mice were as follows: the temperature was 23 ± 1 °C, the humidity was 55 ± 10%, the lights were turned on between 7 am and 7 pm (the lights were turned off at other time), and the mice were allowed to freely take in water and feed. The experimental feed was mouse growth-stable feed (GB M2118), and the daily feeding and management of the animals were under the responsibility of the animal security department, which provided the animals with sufficient diet and fresh drinking water daily.

Experimental grouping: male db/db mice (20 ± 2g) were selected, and 18 male mice in each group were tested. The experimental groups included normal control group (db/m, n = 18), model group (db/db, n = 18), obacunone group (db/db, n = 18), isoobacunoic acid group (db/db, n = 18), obacunone glycoside group (db/db, n = 18), gliclazide group (db/db, n = 18), obacunone/gliclazide combination group (db/db, n = 18), isoobacunoic acid/gliclazide combination group (db/db, n = 18), obacunone glycoside/gliclazide combination group (db/db, n = 18).

Gavage doses: obacunone at a dose of 0.04 g/kg was gavaged per day for the obacunone group, isoobacunoic acid at a dose of 0.04 g/kg was gavaged per day for the isoobacunoic acid group, obacunone glycoside at a dose of 0.04 g/kg was gavaged per day for the obacunone glycoside group, gliclazide at a dose of 0.04 g/kg was gavaged per day for the gliclazide group, obacunone at a dose of 0.02 g/kg and gliclazide at a dose of 0.02 g/kg were simultaneously gavaged per day for the obacunone/gliclazide combination group, isoobacunoic acid at a dose of 0.02 g/kg and gliclazide at a dose of 0.02 g/kg were simultaneously gavaged per day for the isoobacunoic acid/gliclazide combination group, obacunone glycoside at a dose of 0.02 g/kg and gliclazide at a dose of 0.02 g/kg were simultaneously gavaged per day for the obacunone glycoside/gliclazide combination group, the gavage volume was 10 mL/kg, and the normal group and the model group were administrated with 10 mL/kg of distilled water. Two weeks later, the blood glucose values were measured by tail trimming method (Johnson's blood glucose meter) 1 h after the last administration, and serum leptin levels were measured with blood collected from orbital cavity by enzyme-linked immunosorbent assay (Elisa), and the average of each group was obtained. SPSS 16.0 software was used for statistical analysis. The data were expressed as mean and standard deviation. The data before and after were analyzed by t-test, and P <0.05 was considered statistically significant. The test results were shown in Table 2 below.

**Table 2: Blood glucose values and leptin of db/db mice after two weeks of daily gavage.**

| Group | Formulation and dosage of administration | Blood glucose value (mmol/L) | Leptin (pg/ml) |
|---|---|---|---|
| Normal control group | None | 6.0±0.7 | 0.88±0.18 |
| Model group | None | 23.9±4.2 | 48.19+8.12 |
| Obacunone group | Obacunone 0.04g/kg | 12.7±2.8** | 25.99±3.92** |
| Isoobacunoic acid group | Isoobacunoic acid 0.04g/kg | 14.6±3.1** | 27.68±3.25** |
| Obacunone glycoside group | Obacunone glycoside 0.04g/kg | 13.6±2.9** | 28.55±4.00** |
| Gliclazide group | Gliclazide 0.04g/kg | 11.5±2.9** | 26.55±4.32** |
| Obacunone combination group | Obacunone 0.02 g/kg + Gliclazide 0.02 g/kg | 6.5±1.2** | 13.15±3.81 ** |
| Isoobacunoic acid combination group | Isoobacunoic acid 0.02 g/kg + Gliclazide 0.02 g/kg | 6.9±0.9** | 14.55+3.92** |
| Obacunone glycoside combination | Obacunone glycoside 0.02 g/kg + Gliclazide 0.02 g/kg | 6.8±1.1** | 13.87±3.11** |

| | | | |
|---|---|---|---|
| Note **: after independent t-test, compared with the model group, the difference was extremely significant (P <0.01) *: after independent t-test, compared with the model group, the difference was extremely significant (P <0.05) | | | |

### Discussion of experimental results

From the above results, it could be seen that, compared with the model group, either in single administration or in combination administration with gliclazide, obacunone and derivatives thereof could significantly reduce the blood glucose levels in the db/db diabetic mice. When obacunone and derivatives thereof were administrated in combination with gliclazide, significantly improved effect was observed relative to the single administration thereof, showing a synergistic effect. In addition, when obacunone and derivatives thereof were administrated in combination with gliclazide, as compared with their single administration, the doses of both could be effectively reduced while comparable glucose-lowering effects could still be achieved, which improved the safety of therapeutic regimen and reduced side effects.

Meanwhile, the limonoid compound represented by obacunone and its derivatives could significantly improve the sensitivity to leptin; and especially when administrated in combination with gliclazide, it could significantly improve the utilization efficiency of leptin in the body, improve the glucose metabolism of the body, and improve the functions relevant to the glucose metabolism in diabetes mice.

### Example 3

### Effects of limonoid compound, glipizide or combination thereof on blood glucose in mouse pancreatic islet β-cell injury model

In this example, a mouse pancreatic islet β-cell injury model was established by modeling ICR mice with streptozotocin (STZ) (Li Nan et al., Protective effect of pine pollen on kidney damage in diabetic nephropathy mice, Science and Technology Review, 2014, 32 (4/5): 95-99), and used to complete the evaluation of hypoglycemic effect in animals (this model could simulate pancreatic islet β-cell damage state of type I and type II diabetics). The limonoid compound was selected from the group consisting of ichangin, ichangensin, and ichangin glycoside, and glipizide single administration group, ichangin single administration group, ichangensi single administration group, ichangin glycoside singe administration group, and respective combination thereof with glipizide administration groups were set.

Conditions of experimental feeding: ICR mice (20 ± 2g), aged 6 weeks, purchased from Zhejiang Academy of Medical Sciences, and subjected to experimental feeding after 7 days of preliminary feeding. It should be noted that the conditions for raising the mice were as follows: the temperature was 23 ± 1 °C, the humidity was 55 ± 10%, the lights were turned on between 7 am and 7 pm (the lights were turned off at other time), and the mice were allowed to freely take in water and feed. The experimental feed was mouse growth-stable feed (GB M2118), and the daily feeding and management of the animals were under the responsibility of the animal security department, which provided the animals with sufficient diet and fresh drinking water daily.

Experimental grouping: 15 male mice were randomly selected as the normal control group. After fasting for 12 hours, the remaining mice were intraperitoneally injected once with STZ at a dose of 150 mg/kg, and 72 hours later, the mice with blood glucose value of 15 to 25 mmol/L were undifferentiatedly grouped and used in the experiment, 15 animals in each group, and subjected to blood sampling and detection of indicators after two weeks of administration.

Gavage doses: the gavage dose was 0.1 g/kg per day for the ichangin group, the gavage dose was 0.1 g/kg per day for the ichangensin group, the gavage dose was 0.1 g/kg per day for the ichangin glycoside group, the gavage dose of glipizide was 0.1 g/kg per day for the glipizide group, ichangin at a dose of 0.05 g/kg and glipizide at a dose of 0.05 g/kg were simultaneously gavaged per day for the ichangin/glipizide combination group, ichangensin at a dose of 0.05 g/kg and glipizide at a dose of 0.05 g/kg were simultaneously gavaged per day for the ichangensin/glipizide combination group, ichangin glycoside at a dose of 0.05 g/kg and glipizide at a dose of 0.05 g/kg were simultaneously gavaged per day for the ichangin glycoside/glipizide combination group, the gavage volume was 10 mL/kg, and the normal group and the model group were administrated with 10 mL/kg of distilled water. Two weeks later, the blood glucose values were measured by tail trimming method (Johnson's blood glucose meter) 1 h after the last administration, and the average of each group was obtained. SPSS 16.0 software was used for statistical analysis. The data were expressed as mean and standard deviation. The data before and after were analyzed by t-test, and P <0.05 was considered statistically significant. The test results were shown in Table 3 below.

**Table 3: Blood glucose values of STZ mice after two weeks of daily intragastric gavage.**

| Group | Formulation and dosage of administration | Blood glucose value (mmol/L) |
|---|---|---|
| Normal control group | None | 7.5±0.6 |
| Model group | None | 29.5±4.6 |
| Glipizide group | Glipizide 0.1g/kg | 14.5±2.3** |
| Ichangin group | Ichangin 0.1g/kg | 14.8±1.9** |
| Ichangensin group | Ichangensin 0.1g/kg | 14.5±3.5** |
| Ichangin glycoside group | Ichangin glycoside 0.1g/kg | 14.9±2.1** |
| Ichangin combination group | Ichangin 0.05g/kg + Glipizide 0.05 g/kg | 7.6±1.9** |
| Ichangensin combination group | Ichangensin 0.05 g/kg + Glipizide 0.05 g/kg | 7.7±1.2** |
| Ichangin glycoside combination group | Ichangin glycoside 0.05 g/kg + Glipizide 0.05 g/kg | 7.5±1.5** |

| | | |
|---|---|---|
| Note **: After independent t-test, compared with the model group, the difference was extremely significant (P <0.01) *: After independent t-test, compared with the model group, the difference was extremely significant (P <0.05) | | |

### Discussion of experimental results

From the above results, it could be seen that, compared with the model group, either in single administration or in combination administration with glipizide, the three limonoid compounds all could significantly lower the blood glucose levels in the mice of the STZ pancreatic islet cell injury model. When they were administrated in combination with glipizide, their effects were significantly increased as compared with their single administration, similar to the normal mice in blood glucose level, showing a synergistic effect. In addition, when the above three limonoid compounds were administrated in combination with glipizide, as compared with their single administration, the doses of both could be effectively reduced while comparable glucose-lowering effects could still be achieved, which improved the safety of therapeutic regimen and reduced side effects.

### Example 4

### Effects of limonoid compound, gliquidone or combination thereof on blood glucose and insulin in mouse type II diabetes model

In the present embodiment, the limonoid compound was selected from nomilin, deacetylnomilin, nomilin acid, deacetylnomilin acid glycoside, and gliquidone single administration group, nomilin single administration group, deacetylnomilin single administration group, nomilinic acid single administered group, deacetylnomilinic acid glycoside single administration group, and respective combination thereof with gliquidone administration groups were set.

Conditions for experimental feeding: as type II diabetes model mice, 6-week-old SPF-grade db/db mice were purchased from the Nanjing Model Biology Institute, and subjected to experimental feeding after 7 days of preliminary feeding. It should be noted that the conditions for raising the mice were as follows: the temperature was 23 ± 1 °C, the humidity was 55 ± 10%, the lights were turned on between 7 am and 7 pm (the lights were turned off at other time), and the mice were allowed to freely take in water and feed. The experimental feed was mouse growth-stable feed (GB M2118), and the daily feeding and management of the animals were under the responsibility of the animal security department, which provided the animals with sufficient diet and fresh drinking water daily.

Experimental grouping: male db/db mice (20 ± 2g) were selected, 18 male mice in each group were tested, and drinking bottles were sterilized weekly. The experimental groups included normal control group (db/m, n = 18), model group (db/db, n = 18), nomilin group (db/db, n = 18), deacetylnomilin group (db/db, n = 18), nomilinic acid group (db/db, n = 18), deacetylnomilinic acid glycoside group (db/db, n = 18), gliquidone group (db/db, n = 18), nomilin combination group (db/db, n = 18), deacetylnomilin combination group (db/db, n = 18), nomilinic acid combination group (db/db, n = 18), deacetylnomilinic acid glycoside combination group (db/db, n = 18).

Gavage doses: nomilin at a dose of 0.2 g/kg was gavaged per day for the nomilin group, deacetylnomilin at a dose of 0.2 g/kg was gavaged per day for the deacetylnomilin group, nomilinic acid at a dose of 0.2 g/kg was gavaged per day for the nomilinic acid group, deacetylnomilinic acid glycoside at a dose of 0.2 g/kg was gavaged per day for the deacetylnomilinic acid glycoside group, gliquidone at a dose of 0.2 g/kg was gavaged per day for the gliquidone group, nomilin at a dose of 0.1 g/kg and gliquidone at a dose of 0.1 g/kg were simultaneously gavaged per day for the nomilin combination group, nomilinic acid at a dose of 0.1 g/kg and gliquidone at a dose of 0.1 g/kg were simultaneously gavaged per day for the nomilinic acid combination group, deacetylnomilin at a dose of 0.1 g/kg and gliquidone at a dose of 0.1 g/kg were simultaneously gavaged per day for the deacetylnomilin combination group, deacetylnomilinic acid glycoside at a dose of 0.1 g/kg and gliquidone at a dose of 0.1 g/kg were simultaneously gavaged per day for the deacetylnomilinic acid glycoside combination group, the gavage volume was 10 mL/kg, and the normal group and the model group were administrated with 10 mL/kg of distilled water. Two weeks later, the blood glucose values were measured by tail trimming method (Johnson's blood glucose meter) 1 h after the last administration, and serum insulin levels were measured with blood collected from orbital cavity by enzyme-linked immunosorbent assay (Elisa), and the average of each group was obtained. SPSS 16.0 software was used for statistical analysis. The data were expressed as mean and standard deviation. The data before and after were analyzed by t-test, and P <0.05 was considered statistically significant. The test results were shown in Table 4 below.

**Table 4: Blood glucose values and insulin of db/db mice after two weeks of daily gavage.**

| Group | Formulation and dosage of administration | Blood glucose value (mmol/L) | Insulin (pg/ml) |
|---|---|---|---|
| Normal control group | None | 6.0±0.7 | 1.05±0.39 |
| Model group | None | 23.9±4.2 | 10.56±3.00 |
| gliquidone group | gliquidone 0.2g/kg | 10.3±2.1** | 7.82±1.52** |
| Nomilin group | Nomilin acid 0.2g/kg | 10.1±3.9** | 8.82±3.42** |
| Nomilin acid group | Nomilin acid 0.2g/kg | 11.0±4.1** | 8.08±1.49** |
| Deacetylnomilin group | Deacetylnomilin 0.2g/kg | 11.9±2.9** | 8.59±2.61** |
| Deacetylnomilin acid glycoside group | Deacetylnomilin acid glycoside 0.2 g/kg | 12.8±5.5** | 8.40±3.27** |
| Nomilin combination group | Nomilin 0.1 g/kg + gliquidone 0.1 g/kg | 6.2±0.9** | 3.89±0.55** |
| Nomilin acid combination group | Nomilin acid 0.1 g/kg + gliquidone 0.1 g/kg | 6.4±1.1** | 4.99±1.00** |
| Deacetylnomilin combination group | Deacetylnomilin 0.1 g/kg + gliquidone 0.1 g/kg | 6.5±1.1** | 4.97±1.05** |
| Deacetylnomilin acid glycoside combination group | Deacetylnomilin acid glycoside 0.1 g/kg + gliquidone 0.1 g/kg | 6.3±0.9** | 4.68±0.88** |

| | | | |
|---|---|---|---|
| Note **: after independent t-test, compared with the model group, the difference was extremely significant (P <0.01) *: after independent t-test, compared with the model group, the difference was extremely significant (P <0.05) | | | |

### Discussion of experimental results

From the above results, it could be seen that, compared with the model group, either in single administration or in combination administration with gliquidone, nomilin and derivatives thereof could significantly reduce the blood glucose levels in the db/db diabetic mice. When nomilin and derivatives thereof were administrated in combination with gliquidone, significantly improved effect was observed relative to the single administration thereof, showing a synergistic effect. In addition, when nomilin and derivatives thereof were administrated in combination with gliquidone, as compared with their single administration, the doses of both could be effectively reduced while comparable glucose-lowering effects could still be achieved, which improved the safety of therapeutic regimen and reduced side effects.

Meanwhile, the limonoid compound represented by nomilin and derivatives thereof could significantly improve the sensitivity to insulin; and especially when administrated in combination with gliquidone, it could significantly improve the utilization efficiency of insulin in the body, improve the glucose metabolism of the body, and improve the functions relevant to the glucose metabolism in diabetes mice.

### Example 5

### Effects of limonoid compound, glimepiride or combination thereof on blood glucose in mouse type II diabetes model with pancreatic islet damage and obesity

In this example, a mouse model of type II diabetes with pancreatic islet damage and obesity was established by multiple modeling ICR mice with a small dose of streptozotocin (STZ), following with continuous high-fat diets (referring to literature: Zhang Jiyuan et al, Study on the effect of three plant extracts on improving glucose and lipid metabolism in type 2 diabetic mice, Food and Machinery, 2016, 32 (12): 142-147). The limonoid compound was selected from the group consisting of nomilin glycoside, deacetylnomilin glycoside, and nomilinic acid glycoside, and glimepiride single administration group, nomilin glycoside single administration group, deacetylnomilin single administration group, nomilinic acid glycoside single administration group, and respective combination thereof with glimepiride administration groups were set.

Conditions of experimental feeding: ICR mice (20 ± 2g), aged 6 weeks, purchased from Zhejiang Academy of Medical Sciences, and subjected to experimental feeding after 7 days of preliminary feeding. It should be noted that the conditions for raising the mice were as follows: the temperature was 23 ± 1 °C, the humidity was 55 ± 10%, the lights were turned on between 7 am and 7 pm (the lights were turned off at other time), and the mice were allowed to freely take in water and feed. The experimental feed was mouse growth-stable feed (GB M2118), and the daily feeding and management of the animals were under the responsibility of the animal security department, which provided the animals with sufficient diet and fresh drinking water daily.

Experimental grouping: 15 male mice were randomly selected as the normal control group, and the remaining mice were subjected to a high-fat diet (high-fat diet formula: cholesterol 1%, egg yolk powder 10%, lard oil 10%, and basic feed 79%, for establishing an obesity mouse model) for consecutive 4 weeks and intraperitoneal injection of STZ at a dose of 35mg/kg for three consecutive days. After one week, the mice were subject to 24 hours of fasting and water deprivation, their fasting blood glucose was measured, and the mice with a blood glucose level of 15 to 25 mmol/L were selected and undifferentiatedly grouped and used in the experiment, continuously subjected to the high-fat diet, 15 mice in each group, and subjected to blood sampling and detection of indicators after 2 weeks of administration.

Gavage doses: the gavage dose was 0.5 g/kg per day for the nomilin glycoside group, the gavage dose was 0.5 g/kg per day for the deacetylnomilin glycoside group, the gavage dose was 0.5 g/kg per day for the nomilinic acid glycoside group, glimepiride at a dose of 0.5 g/kg was gavaged per day for the glimepiride group, nomilin glycoside at a dose of 0.25 g/kg and glimepiride at a dose of 0.25 g/kg were simultaneously gavaged per day for the nomilin glycoside/glimepiride combination group, deacetylnomilin glycoside at a dose of 0.25 g/kg and glimepiride at a dose of 0.25 g/kg were simultaneously gavaged per day for the deacetylnomilin glycoside/glimepiride combination group, nomilin acid glycoside at a dose of 0.25 g/kg and glimepiride at a dose of 0.25 g/kg were simultaneously gavaged per day for the nomilinic acid glycoside/glimepiride combination group, the gavage volume was 10 mL/kg, and the normal group and the model group were administrated with 10 mL/kg of distilled water. Two weeks later, the blood glucose values were measured by tail trimming method (Johnson's blood glucose meter) 1 h after the last administration, and the average of each group was obtained. SPSS 16.0 software was used for statistical analysis. The data were expressed as mean and standard deviation. The data before and after were analyzed by t-test, and P <0.05 was considered statistically significant. The test results were shown in Table 5 below.

**Table 5: Blood glucose values of STZ mice after two weeks of daily intragastric gavage.**

| Group | Formulation and dosage of administration | Blood glucose value (mmol/L) |
|---|---|---|
| Normal control group | None | 5.9±0.54 |
| Model group | None | 29.6±6.0 |
| Glimepiride group | Glimepiride 0.5g/kg | 10.4±2.1** |
| Nomilin glycoside group | Nomilin glycoside 0.5g/kg | 12.8±2.9** |
| Deacetylnomilin glycoside group | Deacetylnomilin glycoside 0.5g/kg | 12.7±2.5** |
| Nomilin acid glycoside group | Nomilin acid glycoside 0.5g/kg | 13.5±2.4** |
| Nomilin glycoside combination group | Nomilin glycoside 0.25g/kg + Glimepiride 0.25 g/kg | 6.1±0.7** |
| Deacetylnomilin glycoside combination group | Deacetylnomilin glycoside 0.25 g/kg + Glimepiride 0.25 g/kg | 5.8±0.8** |
| Nomilin acid glycoside combination group | Nomilin acid glycoside 0.25 g/kg + Glimepiride 0.25 g/kg | 6.1±1.2** |

| | | |
|---|---|---|
| Note **: After independent t-test, compared with the model group, the difference was extremely significant (P < 0.01) *: After independent t-test, compared with the model group, the difference was extremely significant (P <0.05) | | |

### Discussion of experimental results

From the above results, it could be seen that, compared with the model group, either in single administration or in combination administration with glimepiride, the three limonoid glycosides all could significantly lower the blood glucose levels in the mice of the STZ type II diabetes model. When they were administrated in combination with glimepiride, their effects were significantly increased as compared with their single administration, similar to the normal mice in blood glucose level, showing a synergistic effect. In addition, when the above three limonoid glycosides were administrated in combination with glimepiride, as compared with their single administration, the doses of both could be effectively reduced while comparable glucose-lowering effects could still be achieved, which improved the safety of therapeutic regimen and reduced side effects.

### Example 6

### Method for preparing a tablet containing combination product of nomilin and glibenclamide

In this example, a method for preparing a tablet of a combination product (nomilin and glibenclamide) of the present invention was exemplarily provided. A single tablet contained the following ingredients: 50 mg of nomilin, 400 mg of glibenclamide hydrochloride, 20 mg of hydroxypropylmethylcellulose, 30 mg of sodium carboxymethylcellulose, and 20 mg of microcrystalline cellulose, 5.2 mg of magnesium stearate, 20.8 mg of Opadry, and there were a total of 1000 tablets.

The preparation method comprised the following steps:
a) dissolving 50 g of nomilin in 5 L of 50% ethanol;
b) passing the raw and auxiliary materials through 100 mesh sieves, leaving them on standby;
c) weighing 400g of glibenclamide hydrochloride, 20g of hydroxypropylmethylcellulose, 30g of sodium carboxymethylcellulose, and 20g of microcrystalline cellulose, placing in a fluidized bed, and setting an inlet air volume of 500 ± 50 m³/h, an inlet air temperature of 90 ± 5 °C, and a product temperature of 70 ± 5 °C, to perform hot melt granulation;
d) spraying a nomilin solution into the fluidized bed, setting an atomizing pressure of 1.0 ± 0.2 bar, and a spraying speed of 30 ± 10 Hz, to perform one-step granulation;
e) passing the resultant granules through a 1.0 mm round-hole screen to perform dry granulation;
f) adding 5.2g of magnesium stearate and mixing for 5min;
g) tabletting by using a 17 × 8.5 mm oval puncher at a pressure of 15 KN;
h) dissolving 20.8g of Opadry 85F32004 in distilled water at a ratio of 1:4, setting parameters of a coating pan as: bed temperature of 40 ± 2 °C, outlet air temperature of 48 ± 2 °C, atomizing pressure of 0.6 Mpa, pan speed of 7 rpm, spray volume of 120g/min, to complete film coating.

## Claims

1. A combination product, comprising a limonoid compound or a pharmaceutically acceptable salt thereof, and a sulfonylurea compound, wherein the sulfonylurea compound is selected from the group consisting of glibenclamide, gliclazide, glipizide, gliquidone and glimepiride, and the limonoid compound is one or more selected from the group consisting of limonin, isolimonic acid, limonin glycoside, isolimonic acid glycoside, obacunone, isoobacunoic acid, obacunone glycoside, ichangin, ichangensin, ichangin glycoside, nomilin, nomilinic acid, deacetylnomilin, nomilin glycoside, deacetylnomilin glycoside and nomilinic acid glycoside.

2. The combination product according to claim 1, wherein the combination product is in the form of a pharmaceutical composition.

3. The combination product according to claim 1, wherein the limonoid compound or a pharmaceutically acceptable salt thereof, and the sulfonylurea compound are each in the form of a separate preparation.

4. The combination product according to any one of claims 1 to 3, wherein the sulfonylurea compound is in an amount of 50 mg to 2000 mg.

5. The combination product according to any one of claims 1 to 3, wherein the limonoid compound is in an amount of 50 mg to 2000 mg.

6. The combination product according to any one of claims 1 to 3, wherein the combination product further comprises a pharmaceutically acceptable carrier, diluent, or excipient.

7. The combination product according to claim 6, wherein the combination product is in the form of tablet, capsule, granule, syrup, powder, lozenge, sachet, cachet, elixir, suspension, emulsion, solution, aerosol, ointment, cream and injection.

8. A combination product according to any one of Claims 1 to 7 for use as a medicament for the prevention and/or treatment of a disease associated with diabetes and metabolic syndrome.

9. The combination product according to any one of claim 1 to 7 for use according to claim 8, wherein the limonoid compound or a pharmaceutically acceptable salt thereof, and the sulfonylurea compound are administered simultaneously or sequentially.

## Patentansprüche

1. Kombinationsprodukt, umfassend eine Limonoidverbindung oder ein pharmazeutisch akzeptables Salz davon und eine Sulfonylharnstoffverbindung, wobei die Sulfonylharnstoffverbindung ausgewählt ist aus der Gruppe bestehend aus Glibenclamid, Gliclazid, Glipizid, Gliquidon und Glimepirid, und die Limonoidverbindung eine oder mehrere ausgewählt aus der Gruppe bestehend aus Limonin, Isolimonsäure, Limonin-Glykosid, Isolimonsäure-Glykosid, Obacunon, Isoobacunoinsäure, Obacunon-Glykosid, Ichangin, Ichangensin, Ichangin-Glykosid, Nomilin, Nomilinsäure, Deacetylnomilin, Nomilin-Glykosid, Deacetylnomilin-Glykosid und Nomilinsäure-Glykosid ist.

2. Kombinationsprodukt nach Anspruch 1, wobei das Kombinationsprodukt in Form einer pharmazeutischen Zusammensetzung vorliegt.

3. Kombinationsprodukt nach Anspruch 1, wobei die Limonoidverbindung oder ein pharmazeutisch akzeptables Salz davon und die Sulfonylharnstoffverbindung jeweils in Form einer separaten Zubereitung vorliegen.

4. Kombinationsprodukt nach einem der Ansprüche 1 bis 3, wobei die Sulfonylharnstoffverbindung in einer Menge von 50 mg bis 2000 mg vorliegt.

5. Kombinationsprodukt nach einem der Ansprüche 1 bis 3, wobei die Limonoidverbindung in einer Menge von 50 mg bis 2000 mg vorliegt.

6. Kombinationsprodukt nach einem der Ansprüche 1 bis 3, wobei das Kombinationsprodukt ferner einen pharmazeutisch akzeptablen Träger, Verdünnungsmittel oder Hilfsstoff umfasst.

7. Kombinationsprodukt nach Anspruch 6, wobei das Kombinationsprodukt in Form von Tabletten, Kapseln, Granulat, Sirup, Pulver, Lutschtabletten, Beuteln, Cachets, Elixieren, Suspensionen, Emulsionen, Lösungen, Aerosolen, Salben, Cremes und Injektionen vorliegt.

8. Kombinationsprodukt nach einem der Ansprüche 1 bis 7 zur Verwendung als Medikament zur Vorbeugung und/oder Behandlung einer mit Diabetes und metabolischem Syndrom verbundenen Krankheit.

9. Kombinationsprodukt nach einem der Ansprüche 1 bis 7 zur Verwendung nach Anspruch 8, wobei die Limonoidverbindung oder ein pharmazeutisch akzeptables Salz davon und die Sulfonylharnstoffverbindung gleichzeitig oder nacheinander verabreicht werden.

## Revendications

1. Produit combiné, comprenant un composé limonoïde ou un sel pharmaceutiquement acceptable de celui-ci, et un composé sulfonylurée, dans lequel le composé sulfonylurée est choisi dans le groupe consistant en glibenclamide, gliclazide, glipizide, gliquidone et glimépiride, et le composé limonoïde est l'un ou plusieurs des composés choisis dans le groupe consistant en limonine, acide isolimonique, limonine glycoside, acide isolimonique glycoside, obacunone, acide iso-obacunoïque, obacunone glycoside, ichangine, ichangensine, ichangine glycoside, nomiline, acide nomilinique, désacétylnomiline, nomiline glycoside, désacétylnomiline glycoside et acide nomilinique glycoside.

2. Produit combiné selon la revendication 1, le produit combiné étant sous la forme d'une composition pharmaceutique.

3. Produit combiné selon la revendication 1, dans lequel le composé limonoïde ou un sel pharmaceutiquement acceptable de celui-ci, et le composé sulfonylurée sont chacun sous la forme d'une préparation séparée.

4. Produit combiné selon l'une quelconque des revendications 1 à 3, dans lequel le composé sulfonylurée est en une quantité de 50 mg à 2000 mg.

5. Produit combiné selon l'une quelconque des revendications 1 à 3, dans lequel le composé limonoïde est en une quantité de 50 mg à 2000 mg.

6. Produit combiné selon l'une quelconque des revendications 1 à 3, le produit combiné comprenant en outre un véhicule, diluant ou excipient pharmaceutiquement acceptable.

7. Produit combiné selon la revendication 6, le produit combiné étant sous la forme de comprimés, capsules, granulés, sirops, poudres, pastilles, sachets, cachets, élixirs, suspensions, émulsions, solutions, aérosols, pommades, crèmes et injections.

8. Produit combiné selon l'une quelconque des revendications 1 à 7 pour l'utilisation en tant que médicament pour la prévention et/ou le traitement d'une maladie associée au diabète et aux syndromes métaboliques.

9. Produit combiné selon l'une quelconque des revendications 1 à 7 pour l'utilisation selon la revendication 8, dans lequel le composé limonoïde ou un sel pharmaceutiquement acceptable de celui-ci, et le composé sulfonylurée sont administrés simultanément ou séquentiellement.
